# EUROPEAN PATENT SPECIFICATION

(11) **EP 1 646 670 B1**
(45) Date of publication and mention of the grant of the patent: **26.02.2014**
(21) Application number: 04777316.3
(22) Date of filing: 29.06.2004
(51) Int. Cl.: C08G 63/08, C08G 63/91, C08L 67/04, C08J 9/26, A61L 26/00, A61L 24/04

(54) **SELF-CROSSLINKABLE POLY(CAPROLACTONE FUMARATE)**
SELBSTVERNETZBARES POLY(CAPROLACTON FUMARAT)
POLY(CAPROLACTONE FUMARATE) A RETICULATION SPONTANEE

(30) Priority: 01.07.2003 US 484620 P
(43) Date of publication of application: 19.04.2006
(73) Proprietor: Mayo Foundation For Medical Education And Research, Rochester, MN 55905 (US)
(72) Inventor: YASZEMSKI, Michael, J., Rochester, MN 55902 (US); CURRIER, Bradford, L., Rochester, MN 55902 (US); JABBARI, Esmaiel, Rochester, MN 55902 (US); LU, Lichun, Rochester, MN 55901 (US)
(74) Representative: Coehn, Markus
(86) International application number: PCT/US2004/021040
(87) International publication number: WO 2005/004811

(56) References cited:
- GB-A- 1 394 396
- US-A- 4 082 816
- US-A- 4 722 948
- US-A- 5 747 605
- US-A- 6 124 373
- US-A- 6 153 664
- US-A1- 2002 028 189
- STOREY R.F., WIGGINS J.S., PUCKETT A.D.: "Bioabsorbale composites . I fundamental design considerations using free radically crosslinkable matrices" POLYMER COMPOSITES, vol. 14, no. 1, 1993, pages 7-16, XP002471235
- PETER S J ET AL: "CROSSLINKING CHARACTERISTICS OF AN INJECTABLE POLY(PROPYLENE FUMARATE)BETA-TRICALCIUM PHOSPHATE PASTE AND MECHANICAL PROPERTIES OF THE CROSSLINKED COMPOSITE FOR USE AS A BIODEGRADABLE BONE CEMENT" JOURNAL OF BIOMEDICAL MATERIALS RESEARCH, WILEY, NEW YORK, NY, US, vol. 44, no. 3, 5 March 1999 (1999-03-05), pages 314-321, XP001069596 ISSN: 0021-9304
- LANG M ET AL: "Functionalized multiarm poly(-caprolactone)s: Synthesis, structure analysis, and network formation" JOURNAL OF APPLIED POLYMER SCIENCE, JOHN WILEY AND SONS INC. NEW YORK, US, vol. 86, no. 9, 28 November 2002 (2002-11-28), pages 2296-2306, XP002275969 ISSN: 0021-8995
- XIE D -L ; GE H -X ; ZHANG L -X ; YANG CH -ZH: "Synthesis and characterization of novel carboxyl telechelic microspheres" JOURNAL OF APPLIED POLYMER SCIENCE, vol. 68, no. 2, 1998, pages 205-216, XP002471236
- STOREY ROBSON F ; WIGGINS JEFFREY S ; TISACK MONICA E ; MAURITZ KENNETH A ; PUCKETT AARON D: "Synthesis and free radical curing of poly(epsilon -caprolactone-co-D,L-lactide) fumarate" POLYMER PREPRINTS, DIVISION OF POLYMER CHEMISTRY, AMERICAN CHEMICAL SOCIETY, vol. 32, no. 1, 1991, pages 629-630, XP009096722
- HAN YANG-KYOO ; EDELMAN PETER G ; HUANG SAMUEL J: "Synthesis and characterization of crosslinked polymers for biomedical composites" JOURNAL OF MACROMOLECULAR SCIENCE ; CHEMISTRY, vol. A25, no. 5-7, 1988, pages 847-869, XP002471237

## Description

### BACKGROUND OF THE INVENTION

### 1. Field of the Invention

This invention relates to the synthesis of a poly(caprolactone-fumarate) polymer useful as a biocompatible, bioresorbable, injectable, and in-situ hardening scaffold for tissue engineering applications.

### 2. Description of the Related Art

The clinical needs for bone regeneration are diverse, and there are roughly 1,000,000 patients who have skeletal defects each year in the United States that require bone graft procedures to achieve union. These include applications arising from resection of primary and metastatic tumors, bone loss after skeletal trauma, primary and revision total joint arthroplasty with bone deficiency, spinal arthrodesis, and trabecular voids following osteoporotic insufficiency fractures. Current clinical decision making in the selection, preparation and application of bone graft materials often involves many factors. From a structural perspective, several decisions need to be addressed prior to deciding on a surgical management plan.

First, the type of bone lost must be determined. The defect may be trabecular bone, cortical bone, or a combination of both structural bone types. Second, the nature of the defect must be defined, whether it is contained and has a bony or soft tissue shell, or is non-contained and represents a segmental loss of bone continuity. Third, the size of the defect (size of trabecular voids or length of segmental defects) must be determined. Mechanical issues that enter into the graft selection decision include the skeletal location of the defect to be reconstructed and the anticipated loads in that location. In addition, biologic issues such as host co-morbidities (for example, diabetes) may all have an effect on the bone graft incorporation process. Finally, surgical issues that play a role in the selection of graft material include consideration regarding the size of the surgical access portal relative to the size of the defect.

Current clinical methods of treating skeletal defects involve bone transplantation or the use of other materials to restore continuity. Autologous bone graft has been the gold standard of bone replacement because it provides such essential elements as osteogenic cells, osteoinductive factors, and an osteoconductive matrix for healing. However, the limited supply of autograft bone, and donor site morbidity both restrict the spectrum of cases in which it can be used alone. Allograft bone, although available in abundant supply, has drawbacks that include reduced rates of graft incorporation compared to autograft bone, and the possibility of pathogen transfer from donor to host.

Metals provide immediate mechanical support at the defect site but exhibit less than ideal overall integration with host tissue and can eventually fail due to fatigue loading if the bone does not heal prior to fatigue failure of the metal. Ceramics, such as β-tricalcium phosphate (β-TCP) and hydroxyapatite are both osteoconductive, and have found clinical use as surface coatings on metal prostheses to enhance bonding of those prostheses to bone. In particulate form, they offer increased mechanical strength to polymeric composite materials primarily in compression, but are less effective in enhancing resistance to torsional and bending forces. Poly(methyl methacrylate) bone cement can be injected or molded and is sometimes used to fill both cavitary and segmental defects, such as those that result from the curettage of a giant cell tumor or from the resection of a vertebral body in metastatic disease to the spine, respectively. However, the temperature can rise up to 100°C during the exothermic polymerization reaction, and the heat released risks local tissue injury. Additionally, poly(methyl methacrylate) is non-biodegradable and can thus accumulate fatigue damage with time and eventually undergo mechanical failure.

Synthetic biodegradable polymers may provide treatment options not currently available. These materials can be manufactured in virtually unlimited supply and the flexibility in their design allows the synthesis of a wide range of polymers with varying mechanical, biologic, degradation, and rheologic properties. For instance, their mechanical and degradation properties can be manipulated by changing the polymer molecular weight during synthesis, and can thus be tailored to fit a particular application. The injectable nature of the skeletal regeneration biomaterial would be ideal to fill defects with limited accessibility or irregular shape. For example, minimally invasive endoscopic techniques now in clinical use would allow the injectable form of the biomaterial to be inserted for posterolateral intertransverse process spinal fusion. This would decrease the surgical trauma from the extensive exposure and muscle stripping that must now be done to put the graft material into position. The injectable material could be placed into cancellous voids from periarticular fractures, osteoporotic spinal fractures, or bone cysts without creating a large access hole in the surrounding cortical bone. These clinical situations represent the motivation for the development of injectable biodegradable polymeric composite materials for bone tissue engineering.

The publication *"*Bioabsorbable Composites: fundamental design considerations using free radically crosslinkable matrices", by Storey, Jeffrey et al. discloses e.g. bioabsorbable composites consisting of poly(glycolic acid) surgical mesh embedded in free radically crosslinked poly(D,L-lactide-co-ε-caprolactone) fumarate or poly(D, L-lactide-co-glycolic acid) fumarate-based matrices.

The document "Functionalized Multiarm Poly(ε-caprolactone)s: synthesis, structure alnalysis and network formation" by Lang and Chu describes the synthesis of four-arm poly(ε-caprolactone)s by ring-opening polymerization by use of pentaerythritol as the center core and their subsequent conversion to maleic acid monoester functionalized poly(ε-caprolactone)s.

The publication "The synthesisand characterization of novel carboxyl thelechelic icrospheres "by Xie, Ge et al. refers to a series of carboxyl telechelic microspheres with different length of oligo-caprolactone telechelic chains.

The publication "Synthesis and characterization of crosslinked polymers for biomedical composites" by Han, Edelman and Huang describes the synthesis of low molecular weight unsaturated polyesters containing C-C double bonds, by the reaction of poly(ε-caprolactone) diol or D, L-lactide and glycolic acid with maleric anhydride or fumaric acid.

Thus, biodegradable scaffolds that can be injected and crosslinked in situ to fill defects offer attractive additions to existing methods (see, Yaszemski et al., "Clinical needs for bone tissue engineering technology", in Bone Engineering, J. E. Davis, Ed. Toronto, Em Squared, 2000, pp. 541-547). Previous materials used as synthetic bone substitute or a synthetic bone cement include poly(methyl methacrylate) as described above, poly(lactide-glycolide) and poly(propylene fumarate). Poly(methyl methacrylate) is not typically bioresorbable. Poly(lactide-glycolide) has a high softening temperature exceeding 150°C and is not crosslinkable. Poly(propylene fumarate) does not become solid upon cooling to human biological temperature of 37°C and it is difficult to control its degradation.

For minimally invasive applications, researchers seek injectable systems that can be crosslinked in situ by chemical redox initiation, can promote tissue growth, and have mechanical properties appropriate for the particular clinical scenario being treated. Although biocompatible polymers that can be injected and crosslinked in situ have been developed for bone tissue engineering, a crosslinking agent such as N-vinyl pyrrolidone (NVP) or methacrylic anhydride in amounts greater than 20% by weight of the macromer is required for crosslinking (see, e.g., Peter et al., "Crosslinking characteristics of an injectable poly(propylene fumarate) I beta-tricalcium phosphate paste and mechanical properties of the crosslinked composite for use as a biodegradable bone cement", J. Biomed. Mater. Res., Vol. 44, pp. 314-321). Crosslinking agents are potentially toxic compounds.

Thus, there is a continuing need for improved biodegradable scaffolds that can be injected and crosslinked in situ to fill bone defects. In particular, there is a need for an improved biocompatible, bioresorbable, and injectable polymer for bone tissue engineering where the polymer can self-crosslink in the absence of a crosslinking agent.

### SUMMARY OF THE INVENTION

In accordance with the invention, a fumaryl halide (e.g., fumaryl chloride) or fumaric acid, which contains unsaturated carbon-carbon double bonds that can be used for in situ crosslinking, is copolymerized with a biodegradable macromer that has a flexible backbone such that the resulting copolymer may self-crosslink in the absence of a crosslinking agent. In particular, the present invention provides a scaffold for tissue regeneration comprising a biodegradable matrix, said matrix comprising an injectable self cross-linkable copolymer, wherein:
(a) the copolymer is a biocompatible, bioresorbable poly(caprolactone fumarate) having a melting point in the range of 50°C to 70°C, prepared by reacting poly(ε-caprolactone) and
   - fumaric acid or a salt thereof or
   - fumaryl chloride
(b) the poly(ε-caprolactone) has a molecular weight in the range of 500 to 10000 Dalton, the molecular weight being the weight average molecular weight calculated according to the formula M_{w}=ΣᵢNᵢMᵢ²/ΣᵢNᵢMᵢ and determined by gel permeation chromatography, and
(c) the copolymer has a number average molecular weight in the range of 3000 to 4000 calculated according to the formula Mₙ=ΣᵢNᵢMᵢ/ΣiNᵢ and determined by gel permeation chromatography.

Additionally, it is also provided a crosslinkable, biodegradable material comprising the above injectable self cross-linkable copolymer and a free radical initiator. Poly (ε-caprolactone) is a degradable polymer approved by the U.S. Food and Drug Administration for use in resorbable sutures, and has excellent biocompatibility. Poly(ε-caprolactone) based constructs have been used for guided bone regeneration (see, Elfick, "Poly(ε-caprolactone) as a potential material for a temporary joint spacer", Biomaterials, Vol. 23, pp. 4463-4467, 2002) and therefore, it is suitable as a flexible macromer for copolymerization with fumaryl chloride. The present invention provides for the synthesis of poly(ε-caprolactone-fumarate) as a biocompatible, bioresorbable, injectable, and self-crosslinkable polymer for bone tissue engineering.

In one embodiment of the invention, a biomaterial is synthesized by reacting fumaryl chloride or fumaric acid with poly(ε-caprolactone) of low molecular weight in the range of 500-10000 daltons to produce poly(ε-caprolactone-fumarate) using the method of condensation polymerization. Low molecular weight poly(ε-caprolactone) can be fabricated into scaffolds by melting or solvent casting but it does not have suitable mechanical properties as a scaffold for regeneration of hard tissues.

The poly(ε-caprolactone-fumarate) which is the subject of this invention is a resorbable and semi-crystalline polymer with a melting point between 50-70 degrees centigrade depending on the molecular weight of the poly(ε-caprolactone-fumarate). Above its melting point, the poly(ε-caprolactone-fumarate) is a free flowing liquid which can be physically mixed with other formulation components such as porogen, initiator, crosslinking agent, accelerator, diluent, foaming, agent, buffering agent, inhibitor catalyst, growth factors, particulate and fiber reinforcing materials, and stabilizers in free or encapsulated form and the poly(ε-caprolactone-fumarate) can be injected via a syringe to fabricate a scaffold used for regeneration of biological tissues. Below the melting point, for example at human biological temperature of 37 degrees centigrade (98.6°F), poly(ε-caprolactone-fumarate) becomes a solid and hardens by physical as well as chemical crosslinking.

Physical crosslinking takes place by partial crystallization of poly(ε-caprolactone) segments of the poly(ε-caprolactone-fumarate) chains. Chemical crosslinking occurs by cross-linking of double bonds of the fumarate groups of poly(ε-caprolactone-fumarate) chains in the presence of suitable initiator, accelerator, or crosslinking agent. However, a crosslinking agent is typically not needed. The extent of physical and chemical crosslinking can be controlled independently by the molecular weight of poly(ε-caprolactone), the molecular weight of the poly(ε-caprolactone-fumarate) macromer, and the ratio of fumarate to poly(ε-caprolactone) in the poly(ε-caprolactone-fumarate) macromer. The degradation behavior of the poly(ε-caprolactone-fumarate) macromer can be also controlled by the molecular weight of poly(ε-caprolactone), the molecular weight of the poly(ε-caprolactone-fumarate) macromer, and the ratio of fumarate to poly(ε-caprolactone) in the poly(ε-caprolactone-fumarate) macromer.

The biocompatible and bioresorbable poly(caprolactone-fumarate) biomaterial according to the invention has a melting point between 50-70 degrees centigrade and a hardening point between 30-40 degrees centigrade. This unique property makes this biomaterial useful in fabrication of injectable and in-situ hardening scaffolds for application in skeletal reconstruction. Application of this invention can be as an injectable bioresorbable synthetic bone substitute or as an injectable bioresorbable bone cement with controlled degradation behavior.

These and other features, aspects, and advantages of the present invention will become better understood upon consideration of the following detailed description, drawings, and appended claims.

### BRIEF DESCRIPTION OF THE DRAWINGS

Figure 1 is a FTIR spectrum of poly(ε-caprolactone-fumarate) with poly(ε-caprolactone) number average molecular weight of 760 daltons.
Figure 2 is a ¹H-NMR spectrum of poly(ε-caprolactone-fumarate) with poly(ε-caprolactone) number average molecular weight of 760 daltons.
Figure 3 is a scanning electron micrograph of the cross-section of a poly(ε-caprolactone-fumarate) scaffold with poly(ε-caprolactone-fumarate) Mₙ of 3680 daltons and polydispersity index of 2.3.
Figure 4 is a micro-computed tomography of a longitudinal section of a poly(ε-caprolactone-fumarate) scaffold with poly(ε-caprolactone-fumarate) Mₙ of 3680 daltons and polydispersity index of 2.3.
Figure 5 is a graph showing the fraction of viable cells after exposure to poly(caprolactone-fumarate) (PCLF) disks for 48 hours.

### DETAILED DESCRIPTION OF THE INVENTION

The invention provides poly(caprolactone fumarate), a biocompatible, bioresorbable, injectable, and self-crosslinkable copolymer that includes caprolactone units as follows 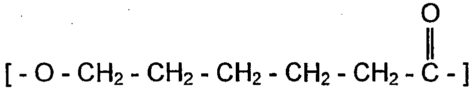 and fumarate units as follows 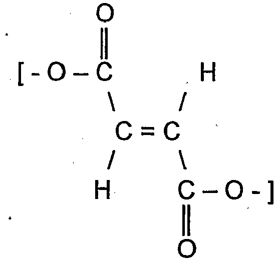 and / or

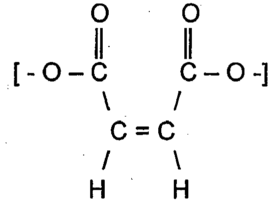

In one form, the copolymer has a number average molecular weight in the range of 3000 to 4000, and a polydispersity index in the range of 2 to 4. The copolymer has a melting point in the range of 50°C to 70°C, and is injectable at temperatures above the melting point.

In one method for preparing the copolymer, a fumaric acid salt such as a fumaryl halide (e.g., fumaryl chloride) or fumaric acid, which contains unsaturated carbon-carbon double bonds that can be used for in situ crosslinking, is copolymerized with a poly(caprolactone) macromer (e.g., poly(ε-caprolactone) having a molecular weight in the range of 500-10000 daltons) that has a flexible backbone such that the resulting copolymer may self-crosslink in the absence of a crosslinking agent.

In another aspect, the invention provides a self-crosslinkable, biodegradable material including a copolymer according to the invention having caprolactone units and fumarate units, and a free radical initiator (e.g., benzoyl peroxide). The self-crosslinkable, biodegradable material may be used as an injectable bone substitute or an injectable bone cement. In one form, the self-crosslinkable, biodegradable material includes a porogen such as sodium chloride crystals. The self-crosslinkable, biodegradable material may also include an accelerator such as dimethyl toluidine. Because the biodegradable material is self-crosslinking, the material does not need to include a crosslinking agent. In another form, the self-crosslinkable, biodegradable material includes particulate or fiber reinforcement materials. Preferably, the particulate or fiber reinforcement materials comprise a bioactive ceramic such as hydroxyapatite.

The self-crosslinkable, biodegradable material may be used to prepare a scaffold for tissue regeneration. The scaffold includes a biodegradable matrix formed from a copolymer according to the invention including caprolactone units and fumarate units. The matrix may include particulate or fiber reinforcement materials such as hydroxyapatite. The scaffold may be formed with a porogen such as crystals of sodium chloride salt or the like to be encapsulated by the polymerizing scaffold and which dissolve upon solidification of the material to form a porous scaffold.

As used herein, a "biocompatible" material is one which stimulates only a mild, often transient, implantation response, as opposed to a severe or escalating response. As used herein, a "biodegradable" material is one which decomposes under normal in vivo physiological conditions into components which can be metabolized or excreted. As used herein, a "bioresorbable" material is one that breaks down over a finite period of time due to the chemical/biological action of the body. By "injectable", we mean the copolymer may be delivered to a site by way of a medical syringe. By "self-crosslinkable", we mean the functional groups of a polymer according to the invention may crosslink with the functional groups of the same polymer or another polymer according to the invention without a crosslinking agent that forms crosslinks between the functional groups of a polymer according to the invention and the functional groups of the same or another polymer according to the invention.

The term "molecular weight" in this specification refers to "weight average molecular weight" (M_{w}= ∑ᵢNᵢMᵢ²/∑ᵢNᵢMᵢ). Although weight average molecular weight (M_{w}) can be determined in a variety of ways, with some differences in result depending upon the method employed, it is convenient to employ gel permeation chromatography. As used herein, the term "number average molecular weight" (Mₙ) refers to the total weight of all the molecules in a polymer sample divided by the total number of moles present (Mₙ = ∑ᵢ Nᵢ Mᵢ / ∑ₗNᵢ). Although number average molecular weight can be determined in a variety of ways, with some differences in result depending upon the method employed, it is convenient to employ gel permeation chromatography. As used herein, the term "polydispersity" refers to the ratio of a materials' "weight average molecular weight" divided by its "number average molecular weight" (M_{w} /Mₙ).

### Examples

The following Examples have been presented in order to further illustrate the invention and are not intended to limit the invention in any way.

### A. Synthesis Of Poly(Caprolactone Fumarate) Macromer

Poly(caprolactone fumarate) macromer was synthesized by condensation polymerization of poly(ε-caprolactone) with fumaryl chloride in methylene chloride with triethylamine as the catalyst. All chemicals were purchased from Aldrich (Milwaukee, WI, USA). Methylene chloride and triethylamine were dried and distilled over calcium hydride before the reaction. Fumaryl chloride was purified by distillation at 161°C. Three samples of poly(ε-caprolactone) with number average molecular weight (Mₙ) of 340, 760, and 1200 Daltons were used. The polydispersity indices of the three samples of poly(ε-caprolactone) were 1.7, 1.8, and 1.8, respectively, as determined by gel permeation chromatography (GPC). The samples of poly(ε-caprolactone) were dried under vacuum of less than 5 mm Hg at 60°C for at least 12 hours before the reaction. The molar ratio of fumaryl chloride to poly(ε-caprolactone) was 0.9. A typical reaction for poly(ε-caprolactone) with Mₙ of 760 was as follows.

In a three-neck reaction flask, 40 mmol of poly(ε-caprolactone) was dissolved in 300 ml. of methylene chloride under nitrogen atmosphere. Thirty-six mmol of fumaryl chloride and 72 mmol of triethylamine dissolved in 25 ml of methylene chloride were added dropwise to the reaction with stirring. The reaction vessel was placed in an ice bath to limit the temperature rise of the exothermic reaction. After the addition of fumaryl chloride and triethylamine, the reaction was continued for 24 hours under ambient conditions.

After completion of the reaction, solvent was removed by rotovaporation at ambient temperature and reduced pressure, the residue was dissolved in 500 ml. of anhydrous ethyl acetate, and the by-product triethylamine hydrochloride salt was removed by filtration. Ethyl acetate was removed by vacuum distillation at 30°C. The polymer was redissolved in methylene chloride and precipitated twice in ice cold ethyl ether. The polymer was dried in vacuum (less than 5 mmHg) at ambient temperature for at least 12 hours and stored at -20°C until used.

### B. Gel Permeation Chromatography

Gel permeation chromatography (GPC) was used to determine the molecular weight and polydispersity of the poly(caprolactone-fumarate) macromer. The GPC was carried out with a Waters 717 Plus Autosampler GPC system (Waters, Milford, MA, USA) connected to a model 515 HPLC pump and model 2410 refractive index detector. The columns consisted of a styragel HT guard column (7.8 x 300 mm, Waters) in series with a styragel HR 4E column (7.8 x 300 MM, Waters). Polymer sample (20 µl), at a concentration of 20 mg/ml in tetrahydrofuran, was eluted at a flow rate of 1 ml/min. Monodisperse polystyrene standards (Polysciences, Warrington, PA) with Mₙ of 0.474, 6.69, 18.6 and 38 kD, and polydispersities of less than 1.1, were used to construct the calibration curve.

### C. ¹H NMR and ¹³C NMR

Nuclear Magnetic Resonance (NMR) was used to confirm the presence of the fumarate group in the macromer. ¹H-NMR and ¹³C-NMR spectra were recorded with a Bruker Advance 500 MHz system (Bruker Analytic GmbH, Rheinstetten, Germany) at ambient temperature. For ¹H-NMR, pulse angle, pulse duration, delay time, acquisition time, resolution, and number of scans were 90°, 6 µsec, 7 s, 3 s, 0.17 Hz, and 32, respectively. For ¹³C-NMR, they were 90°, 4 µsec, 5 s, 200 msec, 2.4 Hz, and 2048, respectively. Polymer solutions for NMR were prepared with deuterated chloroform (99.8 atom % Deutertaed, Aldrich) at a concentration of 50 mg/ml containing 1% v/v trimethyl silane (TMS) as the internal standard.

### D. Fourier Transform Infrared Spectroscopy

An FTS-40 Fourier transform infrared spectrophotometer (FTIR) (Bio-Rad, Hercules, CA, USA) was used to measure the absorption of poly(caprolactone-fumarate) in the IR region from 1000 to 4000 cm⁻¹. A thin film of the polymer was cast on a CaF₂ disk (Wilmad Glass, Buena, NJ, USA) with dimensions of 3 mm x 32 mm and the spectrum was collected under a dry nitrogen atmosphere with 16 averaged scans and a resolution of 4 cm⁻¹. A drop of poly(caprolactone-fumarate) in acetone solution (50 mg of poly(caprolactone-fumarate) in 1 ml. of acetone) was placed on the CaF₂ disk and dried at ambient conditions for 30 minutes. It was next dried in vacuum at ambient temperature for 30 minutes, and finally heated to 60°C to remove any residual solvent in the film.

### E. Scaffold Fabrication

Scaffolds were prepared by self-crosslinking of the fumarate carbon-carbon double bonds via free radical polymerization with sodium chloride salt particles as the porogen. Benzoyl peroxide and dimethyl toluidine were used as the free radical initiator and accelerator, respectively. All chemicals used in crosslinking reaction were obtained from Aldrich and were used as received. The size distribution of salt particles, obtained by sieving, was in the range of 100 to 700 µm, with an average of 400 µm. A typical procedure for fabrication of scaffolds was as follows:

One gram of poly(caprolactone-fumarate) was mixed with 5.0 g of porogen in a scintillation vial, corresponding to 75% by volume porosity. Fifty µl of initiator solution (50 mg. of benzoyl peroxide in 250 µl of N-vinyl pyrrolidone) and 40 µl of accelerator solution (60 µl of dimethyltoluidine in 940 µl of methylene chloride) were added and mixed thoroughly. N-vinyl pyrrolidone, in amount less than 2% by weight of poly(caprolactone-fumarate), was used to dissolve the initiator and not as a crosslinking agent. The polymerizing scaffold was transferred into a 5 mm x 18 mm Teflon mold and pressed manually to maximize packing. The mold was placed in a convection oven for 1 hour to facilitate crosslinking. After crosslinking, the mold was cooled to ambient temperature, scaffolds were removed from the mold, and cylindrical specimens with diameter and length of 5 mm by 8 mm were cut with an Isomet low speed saw (Buehler, Lake Bluff, IL, USA). The salt was leached out by placing the scaffolds in distilled water (DW) for 3 days, during which time water changes occurred every 12 hours. The scaffolds were dried in a controlled atmosphere at ambient temperature for 1 day and in a vacuum of less than 5 mmHg for at least 12 hours.

### F. Scanning Electron Microscopy

A cold field-emission scanning electron microscopy (S-4700, Hitachi Instruments Inc., Tokyo, Japan) was used to examine the pore morphology of the scaffolds. Each scaffold was fractured at liquid nitrogen temperature to expose a flat internal section of the scaffold. The sample was mounted on an SEM stub (Ted Pella, Redding, CA, USA) using Hanid-Tak putty (Super Glue Corp., Rancho Cucamonga, CA, USA) and it was painted with colloidal silver to electrically ground the assembly. It was dried overnight in a vacuum dessicator. The assembly was sputter coated with gold-palladium using a Bio-Rad/Polaron E5400 High Resolution sputter coater (Bio-Rad/Polaron, Cambridge, MA) for 30 s. at 90 mA. The samples were viewed with SEM at 5 kV accelerating voltage.

### G. Micro Computed Tomography

A micro-CT scanner was custom-built in the Physiological Imaging Research laboratory at the Mayo Clinic (Rochester, MN, USA) from commercially available components. The scanner generates 3-D images consisting of up to a billion cubic voxels. The specimen was positioned close to the crystal and rotated in several hundred equiangular steps around 360° between each x-ray exposure and its accompanying charge coupled device recording. A comprehensive 3-D image display and analysis program, (Analyze®, Mayo Foundation, Mayo Clinic, Rochester, MN, USA) was employed to quantitatively assess the polymer scaffold microstructure. An operator-selected threshold of intensities segmented images in order to separate voxels representing regions of differing density. Images were then analyzed to obtain volume fractions of each material, to measure interconnectivity, and to obtain size distributions of isolated pores.

### H. Cytotoxicity Evaluation

Fibroblast cells were isolated from the paws of Mongrel dogs. Briefly, the tendon was excised under aseptic conditions and digested using a solution of 0.2% collagenase type III (Worthington Biochemical Corp., Lakewood, NJ, USA) in Dulbecco's Modified Eagle Medium (Gibco, Life Technologies, Gaithersburg, MD, USA) for 24 hours at 37°C on an orbital shaker. The cell suspension was plated on polystyrene flasks in primary media containing 13.4 grams of DMEM and 3.7 g of sodium bicarbonate in 900 ml. of distilled deionized water, 10% v/v fetal bovine serum (FBS), 5% v/v penicillin-streptomycin solution (10,000 units of penicillin and 10 mg of streptomycin per ml). After plating, the suspension was incubated for 12 hours in a 5% CO₂, 95% relative humidity incubator at 37°C. The cells were washed twice with PBX and enzymatically lifted by exposure to 2 ml. of 0.05% trypsin/0.53 mM EDTA for 5 minutes. The suspension was centrifuged and resuspended in primary media for cytotoxicity evaluation.

The cytotoxicity evaluation proceeded by seeding the cells in a 12 well plate at a density of 2x10⁴ cells/cm² in 300 µl/cm² of primary media in the presence of a sterile 5 mm x 0.5 mm poly(caprolactone-fumarate) disk. The plate was incubated for 48 hours for cell attachment and proliferation. The cells were viewed with an Axiovert 25 Zeiss light microscope (Carl Zeiss, Germany). Poly(caprolactone-fumarate) films were prepared, as described in the scaffold fabrication section, by self-crosslinking poly(caprolactone-fumarate) with benzoyl peroxide and dimethyltoluidine as the initiator and accelerator, respectively, without the salt as the porogen. To make films, the mixture was pressed at 40°C with 275 lb/in² of pressure. Disks with diameter of 5 mm. were cut from the films using a cork-borer. The disks were sterilized by transferring to excess 70% ethanol overnight with shaking, and were then washed with PBS at least three times before adding to the 12 well plates.

### I. Cytocompatibility Evaluation

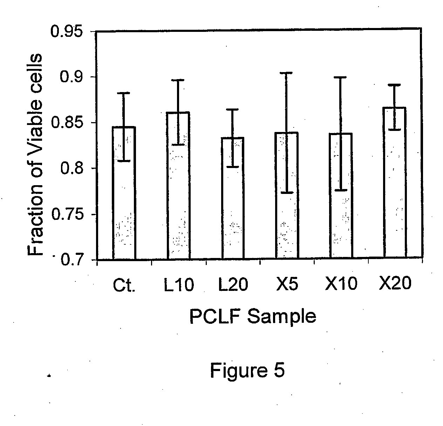

ASTM F813-01 was used for direct contact cell evaluation of the poly(caprolactone-fumarate) (PCLF) scaffolds. Human osteoblast cells were used. Isolation and characterization of the cells is described in S.A. Harris, et al., Development and characterization of a conditionally immortalized human fetal osteoblast cell line, J. Bone. Miner. Res., 10-2 (1995) p. 178-186. The cryopreserved cells were thawed and plated on polystyrene flasks in media containing DMEM/F12 and sodium bicarbonate, 10% v/v fetal bovine serum (FBS), and 150 mg of Geneticin. After plating, the suspension was incubated for 12 hours in a 5% CO₂, 95% relative humidity incubator at 34°C. The cells were seeded in a 24 well plate at a density of 6.5x10⁴ cells/cm² in 300 µl of media and incubated for 48 h. Next, the disks were removed, cells were washed with PBS, fresh media was added to each well, and incubated for an additional 24 hours. The cells were counted with a haemocytometer using a Trypan Blue stain. The fraction of viable cells after exposure to PCLF disks for 48 hours is shown in Figure 5. For the control, linear PCLF with PCL Mₙ of 760 (L10) and 1200 (L20), x-linked PCLF with PCL Mₙ of 340 (X5), 760 (X10), and 1200 g/mol (X20), the fraction of viable cells were 0.85±0.04, 0.86±0.04, 0.83±0.03, 0.84±0.07, 0.84±0.06, and 0.86±0.02, respectively. No adverse effects on the human osteoblast cells were observed in the presence of the PCLF scaffolds.

### Results And Discussion

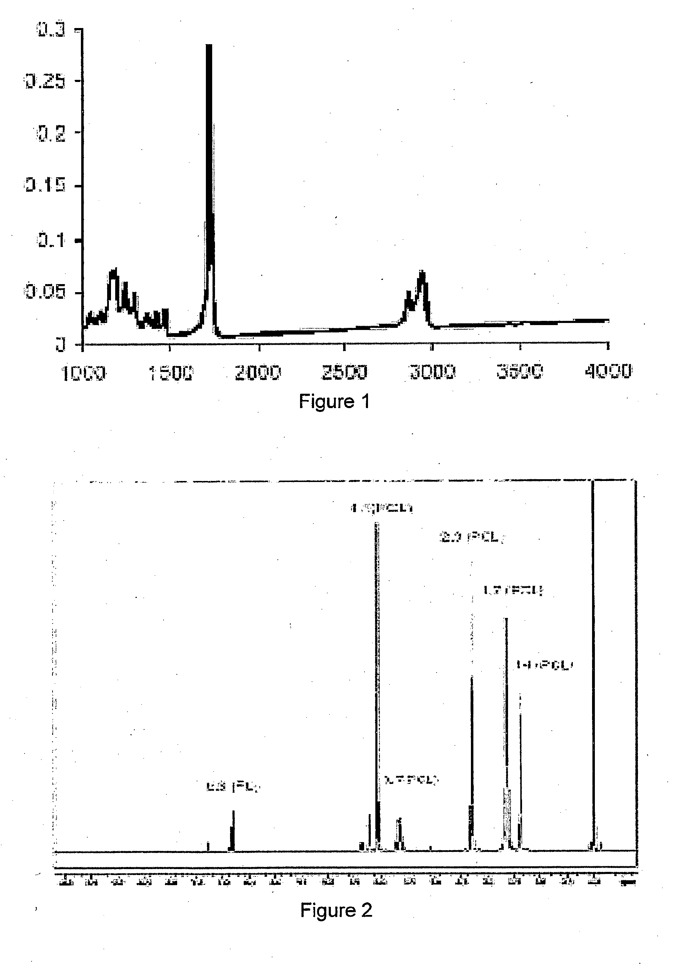

An FTIR spectrum of poly(caprolactone-fumarate) with Mₙ of 3680 daltons with a poly(caprolactone) number average molecular weight of 760 daltons was reviewed. See Figure 1. Absorption bands with peaks positions of 2944 and 2865 cm⁻¹ were observed and are due to the asymmetrical stretching (vₐₛ CH₂) and symmetrical stretching (vₛ CH₂) of the methylene groups of poly(caprolactone). An absorption band with peak position of 1724 cm⁻¹ was observed and is due to the carbonyl (C=O) vibration of poly(caprolactone) and fumarate groups. A weak absorption band was observed with peak position of 1419 cm⁻¹ is due to the C-H rocking vibration of the cis-disubstituted fumarate group, which is absent in the spectra of poly(caprolactone).

The ¹H-NMR spectrum of poly(caprolactone-fumarate) with Mₙ of 3680 daltons with a poly(caprolactone) number average molecular weight of 760 daltons was reviewed. See Figure 2. Chemical shifts with peak positions is 1.38 and 1.65 ppm were observed and are due to methylene hydrogens of poly(caprolactone) attached to two methylene (-CH₂) groups of one methylene group and one (-CH₂O) group, respectively. A chemical shift with peak position at 2.3 ppm was observed and is due to the methylene hydrogens of poly(caprolactone) attached to one (-CH₂) and one carboxyl (-COOR) group. A chemical shift centered at 3.6 ppm was observed and is due to terminal methylene hydrogens of poly(caprolactone) attached to a -CH₂ and a hydroxyl group (-OH). A chemical shift centered at 4.1 ppm was observed and is due to non-terminal methylene hydrogens attached to a -CH₂ and a -OCOR. The ratio of the peak centered at 4.1 ppm to the one at 3.6 ppm, which is equal to 3.9 for poly(caprolactone-fumarate) with Mₙ of 3680 daltons, is related to the degree of copolymerization of poly(caprolactone) with fumaryl chloride. This ratio is consistent with molecular weight data of poly(caprolactone-fumarate) obtained by gel permeation chromatography.

Relatively small peaks centered at 3.1 and 4.35 ppm were observed and are due to the methylene hydrogens of the residual ethoxy groups in poly(caprolactone). A chemical shift with peak position at 6.8 ppm was observed and is due to hydrogens of fumarate group of poly(caprolactone-fumarate), which is absent in the spectrum of poly(caprolactone). Since the chemical shift of the fumarate hydrogens is below 7.0 ppm, the fumarate group in the copolymer is in the cis configuration. The presence of chemical shifts at 6.8 ppm clearly indicated that fumarate groups are incorporated in poly(caprolactone).

The molecular weight of poly(caprolactone-fumarate) (PCLF) as a function of poly(caprolactone) (PCL) molecular weight is shown in Table 1. The fumaryl chloride - poly(caprolactone) (FC/PCL) ratio was 0.9 for all formulations. Poly(caprolactone) number average molecular weights of 340, 760, and 1200 daltons produced poly(caprolactone-fumarate) copolymers with molecular weights of 3150, 3680, and 3870 daltons respectively. For all molecular weights, the polydispersity index of poly(caprolactone-fumarate) was significantly higher than that of poly(caprolactone).

**Table 1. Number average (Mₙ) and polydispersity index (PI) of the synthesized poly(caprolactone-fumarate) polymer with fumaryl chloride - poly(caprolactone) molar ratio of 0.9**

| Sample | FC/PCL Ratio | PCL | | PCLF | |
|---|---|---|---|---|---|
| | | Mₙ | PI | Mₙ | PI |
| 1 | 0.9 | 340 ± 10 | 1.7 ± 0.1 | 3150 ± 90 | 2.9 ± 0.1 |
| 2 | 0.9 | 760 ± 60 | 1.8 ± 0.1 | 3680 ± 20 | 2.3 ± 0.1 |
| 3 | 0.9 | 1200 ± 50 | 1.8 ± 0.1 | 3870 ± 110 | 2.6 ± 0.1 |

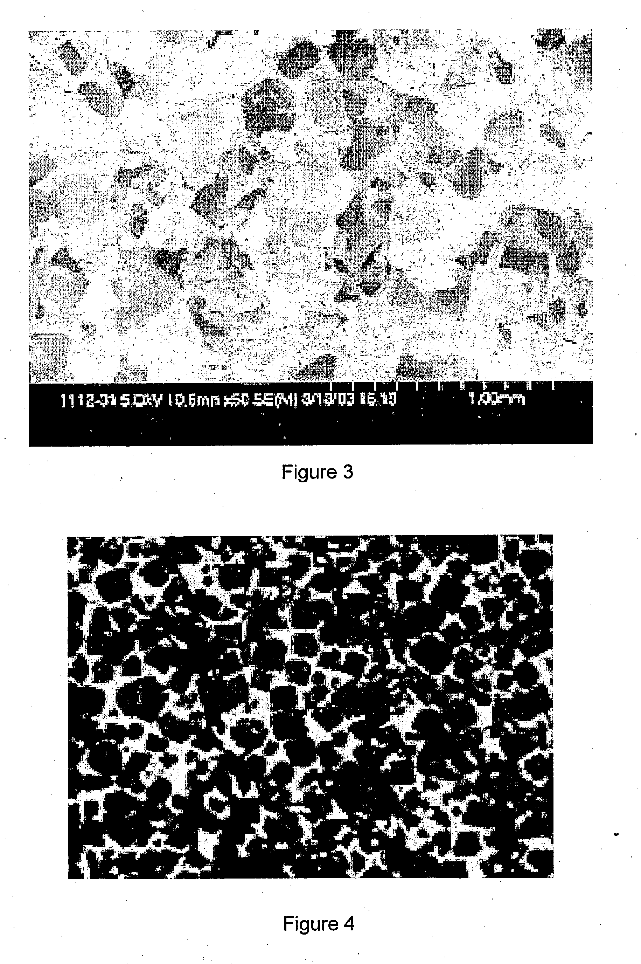

Scaffolds were fabricated from the poly(caprolactone-fumarate) copolymer with salt as the porogen, benzoyl peroxide as the chemical initiator, and dimethyltoluidine as the accelerator. An SEM photograph of a cross-section of the poly(caprolactone-fumarate) scaffold with Mₙ of 3680 daltons, polydispersity index of 2.3, and 75% by volume salt content was prepared. See Figure 3. The SEM picture showed a highly porous and interconnected solid scaffold.

A longitudinal section of the same scaffold, obtained by micro-CT, was also prepared. See Figure 4. The micro-computed tomography picture confirmed the formation of a three-dimensional porous and interconnected poly(caprolactone-fumarate) scaffold in the absence of a crosslinking agent.

Disks of poly(caprolactone-fumarate) self-crosslinked with chemical initiation were exposed to cultured fibroblast cells for up to 48 hours. Cells proliferated in the presence of the poly(caprolactone-fumarate) disks. No adverse effects on the cells were observed in the presence of the poly(caprolactone-fumarate) scaffolds.

Thus, a self-crosslinkable and biodegradable macromer has been developed for guided bone regeneration. In one embodiment, the macromer is a copolymer of fumaryl chloride, which contains double bonds for in-situ crosslinking, and poly(ε-caprolactone) that has a flexible chain to facilitate self-crosslinkability. The poly(caprolactone-fumarate) was characterized with Fourier transform infrared spectroscopy, nuclear magnetic resonance spectroscopy, and get permeation chromatography. Porous scaffolds were fabricated with sodium chloride particles as the porogen and a chemical initiation system. The poly(caprolactone-fumarate) scaffolds were characterized with scanning electron microscopy and micro-computed tomography. The poly(caprolactone-fumarate) copolymer was successfully self-crosslinked without the use of a crosslinking agent. This copolymer is useful as an injectable self-crosslinkable material to treat skeletal defects.

### INDUSTRIAL APPLICABILITY

The present invention relates to crosslinkable, biodegradable polymeric materials that can be injected and then hardened in situ to form scaffolds for tissue and/or skeletal reconstruction.

## Claims

1. A scaffold for tissue regeneration comprising a biodegradable matrix, said matrix comprising an injectable self cross-linkable copolymer, wherein:
(a) the copolymer is a biocompatible, bioresorbable poly(caprolactone fumarate) having a melting point in the range of 50°C to 70°C, prepared by reacting poly(ε-caprolactone) and
- fumaric acid or a salt thereof or
- fumaryl chloride
(b) the poly(ε-caprolactone) has a molecular weight in the range of 500 to 10000 Dalton, the molecular weight being the weight average molecular weight calculated according to the formula M_{w}=ΣᵢNᵢMᵢ²/ΣᵢNᵢMᵢ and determined by gel permeation chromatography, and
(c) the copolymer has a number average molecular weight in the range of 3000 to 4000 calculated according to the formula Mₙ=ΣᵢNᵢMᵢ/ΣᵢNᵢ and determined by gel permeation chromatography.

2. The scaffold of claim 1 wherein the copolymer has a
polydispersity index in the range of 2 to 4.

3. The scaffold of claim 1 wherein the copolymer is injectable at temperatures above the melting point.

4. The scaffold of claim 1 wherein the copolymer has a hardening point in the range of 30°C to 40°C.

5. The scaffold of claim 1 wherein the matrix includes
- particulate or
- fiber reinforcement materials.

6. The scaffold of claim 5 wherein the reinforcement materials comprise hydroxyapatite.

7. The scaffold of claim 1 wherein the scaffold is porous.

8. A crosslinkable, biodegradable material comprising; a copolymer according to claim 1 and a free radical initiator.

9. The material of claim 8 wherein the material is an injectable bone substitute or injectable bone cement.

10. The material of claim 8 further comprising: a porogen or an accelerator.

11. The material of claim 8 wherein the material does not include a crosslinking agent.

12. The material of claim 8 further comprising particulate or fiber reinforcement materials.

13. The material of claim 8 wherein the reinforcement materials comprise hydroxyapatite.

## Patentansprüche

1. Ein Gerüst zur Regeneration von Gewebe, das eine biologisch abbaubare Matrix umfasst, wobei besagte Matrix ein injezierbares, sich selbst quervernetzendes Copolymer umfasst, wobei:
(a) das Copolymer ein biokompatibles, bioresorbierbares poly(Caprolactonfumarat) ist, das einen Schmelzpunkt im Bereich zwischen 50°C und 70°C besitzt und hergestellt wird durch das Reagieren eines poly(ε-Caprolacton) und
- Fumarsäure oder ein Salz davon, oder
- Fumarylchlorid
(b) das poly(ε-Caprolacton) ein Molekulargewicht im Bereich von 500 bis 10000 Dalton besitzt, wobei das Molekulargewicht das gewichtsmittlere Molekulargewicht ist, das gemäß der Formel M_{w}=∑ᵢNᵢMᵢ²/∑ᵢNᵢMᵢ berechnet wird, und das durch Gelpermeations-Chromatographie bestimmt wird, und
(c) das Copolymer ein zahlenmittleres Molekulargewicht im Bereich von 3000 bis 4000 hat, das gemäß der Formel Mₙ=∑ᵢNᵢMᵢ/∑ᵢNᵢ berechnet wird, und das durch Gelpermeations-Chromatographie bestimmt wird.

2. Das Gerüst von Anspruch 1, wobei das Copolymer einen Polydispersionsindex im Bereich von 2 bis 4 hat.

3. Das Gerüst von Anspruch 1, wobei das Copolymer bei Temperaturen oberhalb des Schmelzpunktes injezierbar ist.

4. Das Gerüst von Anspruch 1, wobei das Copolymer einen Erhärtungspunkt im Bereich von 30°C bis 40°C hat.

5. Das Gerüst von Anspruch 1, wobei die Matrix enthält:
- teilchenförmige oder
- faserförmige Verstärkungsmaterialien

6. Das Gerüst von Anspruch 5, wobei die Materialien zur Verstärkung Hydroxyapatit umfassen.

7. Das Gerüst von Anspruch 1, wobei das Gerüst porös ist.

8. Ein quervernetzbares, biologisch abbaubares Material umfassend: ein Copolymer gemäß Anspruch 1 und einen freien Radikalstarter.

9. Das Material von Anspruch 8, wobei das Material ein injezierbarer Knochenersatz oder injezierbarer Knochenzement ist.

10. Das Material von Anspruch 8 weiter umfassend: ein Poren generierendes Material oder einen Beschleuniger.

11. Das Material von Anspruch 8, wobei das Material keinen Stoff zur Quervernetzung enthält.

12. Das Material von Anspruch 8 weiter umfassend: teilchenförmige oder faserförmige Verstärkungsmaterialien.

13. Das Material von Anspruch 8, wobei die Verstärkungsmaterialien Materialien Hydroxyapatit umfassen.

## Revendications

1. Échafaudage pour la régénération de tissu comprenant une matrice biodégradable, ladite matrice comprenant un copolymère réticulable auto-injectable, dans lequel :
(a) le copolymère est un poly(fumarate de caprolactone) biocompatible, biorésorbable ayant un point de fusion dans la plage de 50 °C à 70 °C, préparé par réaction de poly(ε-caprolactone) et
- acide fumarique ou un sel de celui-ci ou
- chlorure de fumaryle
(b) la poly(ε-caprolactone) a un poids moléculaire dans la plage de 500 à 10000 daltons, le poids moléculaire étant le poids moléculaire moyen en poids calculé selon la formule Mw = EiNiMi2/EiNiMi, et déterminé par chromatographie par perméation de gel, et
(c) le copolymère a un poids moléculaire moyen en nombre dans la plage de 3000 à 4000 calculé selon la formule Mn = ∑iNiMi/∑iNi et déterminé par chromatographie par perméation de gel.

2. Échafaudage de la revendication 1 dans lequel le copolymère a un indice de polydispersité dans la plage de 2 à 4.

3. Échafaudage de la revendication 1 dans lequel le copolymère est injectable à des températures au-dessus du point de fusion.

4. Échafaudage de la revendication 1 dans lequel le copolymère a un point de durcissement dans la plage de 30 °C à 40 °C.

5. Échafaudage de la revendication 1 dans lequel la matrice comprend
- des matériaux particulaires ou
- de renforcement de fibre.

6. Échafaudage de la revendication 5 dans lequel les matériaux de renforcement comprennent de l'hydroxyapatite.

7. Échafaudage de la revendication 1 dans lequel l'échafaudage est poreux.

8. Matériau réticulable, biodégradable comprenant : un copolymère selon la revendication 1 et un initiateur de radicaux libres.

9. Matériau de la revendication 8 dans lequel le matériau est un substitut d'os injectable ou un ciment osseux injectable.

10. Matériau de la revendication 8 comprenant en outre : un agent porogène ou accélérateur.

11. Matériau de la revendication 8 dans lequel le matériau ne comprend pas un agent de réticulation.

12. Matériau de la revendication 8 comprenant en outre des matériaux de renforcement particulaires ou fibreux.

13. Matériau de la revendication 8 dans lequel les matériaux de renforcement comprennent de l'hydroxyapatite.
